# EUROPÄISCHE PATENTANMELDUNG

(11) **EP 1 295 589 A1**
(43) Veröffentlichungstag der Anmeldung: **26.03.2003**
(21) Anmeldenummer: 02018429.7
(22) Anmeldetag: 16.08.2002
(51) Int. Cl.: A61K 7/02, A61K 7/06, A61K 7/48

(54) **Selbstschäumende, schaumförmige, nachschäumende oder schäumbare kosmetische oder dermatologische Zubereitungen mit einem Gehalt an nichtionischen polymeren Verdickern**

(30) Priorität: 22.09.2001 DE 10146761
(71) Anmelder: Beiersdorf Aktiengesellschaft, 20245 Hamburg (DE)
(72) Erfinder: Riedel, Heidi, 22529 Hamburg (DE); Bleckmann, Andreas, 22926 Ahrensburg (DE); Kröpke, Rainer, 22869 Schenefeld (DE)

(57) **Zusammenfassung**

Selbstschäumende, schaumförmige, nachschäumende oder schäumbare kosmetische und dermatologische Zubereitungen, welche mindestens einen nicht-ionischen polymeren Verdicker gewählt aus der Gruppe der Copolymere aus
(a) Polyethylenglykol der Formel HO-CH₂(CH₂-O-CH₂)ₙ-CH₂-OH, worin n eine ganze Zahl von 100 bis 250 bedeutet,
(b) Polyoxyethylenglykolalkylether der Formel HO(CH₂CH₂O)ₓR¹, worin x eine ganze Zahl von 1 bis 100 und R¹ einen verzweigten oder unverzweigten, gesättigten oder ungesättigten Alkylrest mit 4 bis 18 Kohlenstoffatomen darstellen, und optional
(c) Tetramethoxymethylglycoluril
enthalten.

## Beschreibung

Die vorliegende Erfindung betrifft selbstschäumende, schaumförmige, nachschäumende oder schäumbare kosmetische und dermatologische Zubereitungen, insbesondere hautpflegende kosmetische und dermatologische Zubereitungen.

Schäume bzw. schaumförmige Zubereitungen gehören zu den dispersen Systemen.

Das bei weitem wichtigste und bekannteste disperse System stellen Emulsionen dar. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, daß die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, daß sie eine feine Verteilung von Wirkstoffen auf der Haut erlauben. Allerdings sind kosmetische Schäume in der Regel nur durch Verwendung besonderer Tenside, welche darüberhinaus oft wenig hautverträglich sind, zu erreichen.

Ein Nachteil des Standes der Technik ist es, daß derartige Schäume nur wenig stabil sind, weshalb sie üblicherweise innerhalb von etwa 24 Stunden zusammenfallen. Eine Anforderung an kosmetische Zubereitungen ist aber, daß diese eine möglichst jahrelange Stabilität besitzen. Diesem Problem wird im allgemeinen dadurch Rechnung getragen, daß der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterläßt einen Schaum.

Auch nachschäumende kosmetische Zubereitungen sind an sich bekannt. Sie werden zunächst in fließförmiger Form aus einem Aerosolbehälter auf die Haut aufgetragen und entwickeln nach kurzer Verzögerung erst dort unter dem Einfluß des enthaltenen Nachschäummittels den eigentlichen Schaum, beispielsweise einen Rasierschaum. Nachschäumende Zubereitungen liegen oft in speziellen Ausführungsformen wie etwa nachschäumenden Rasiergelen oder dergleichen vor.

Allerdings kennt der Stand der Technik keinerlei kosmetische oder dermatologische Zubereitungen, welche bereits bei der Herstellung aufgeschäumt werden könnten und dennoch eine genügend hohe Stabilität aufweisen, um in üblicher Weise verpackt, gelagert und in den Handel gebracht zu werden.

Eine Aufgabe der vorliegenden Erfindung war also, den Stand der Technik zu bereichern und kosmetische oder dermatologische selbstschäumende und/oder schaumförmige Zubereitungen zur Verfügung zu stellen, die die Nachteile des Standes der Technik nicht aufweisen.

Die Deutsche Offenlegungsschrift DE 197 54 659 offenbart, daß Kohlendioxid ein geeigneter Wirkstoff zur Stabilisierung oder Erhöhung der epidermalen Ceramidsyntheserate ist, welcher der Stärkung der Permeabilitätsbarriere, der Verminderung des transepidermalen Wasserverlusts und der Steigerung der relativen Hautfeuchtigkeit dienen kann. Zur Behandlung der Haut wird das CO₂ beispielsweise in Wasser gelöst, mit welchem anschließend die Haut gespült wird. Allerdings kennt der Stand der Technik bislang keinerlei kosmetische oder dermatologische Grundlagen, in die ein gasförmiger Wirkstoff in ausreichender, d. h. wirksamer Konzentration eingearbeitet werden könnte.

Eine weitere Aufgabe der vorliegenden Erfindung war es also, kosmetische oder dermatologische Grundlagen zu finden, in die sich wirksame Mengen an gasförmigen Wirkstoffen einarbeiten lassen.

Es war überraschend und für den Fachmann nicht vorauszusehen, daß
selbstschäumende, schaumförmige, nachschäumende oder schäumbare kosmetische und dermatologische Zubereitungen, welche mindestens einen nicht-ionischen polymeren Verdicker gewählt aus der Gruppe der Copolymere aus
(a) Polyethylenglykol der Formel HO-CH₂(CH₂-O-CH₂)ₙ-CH₂-OH, worin n eine ganze Zahl von 100 bis 250 bedeutet,
(b) Polyoxyethylenglykolalkylether der Formel HO(CH₂CH₂O)ₓR¹, worin x eine ganze Zahl von 1 bis 100 und R¹ einen verzweigten oder unverzweigten, gesättigten oder ungesättigten Alkylrest mit 4 bis 40 Kohlenstoffatomen darstellen, und optional
(c) Tetramethoxymethylglycoluril
enthalten,
den Nachteilen des Standes der Technik abhelfen.

Unter "selbstschäumend", "schaumförmig", "nachschäumend" bzw. "schäumbar" ist im Sinne der vorliegenden Erfindung zu verstehen, daß die Gasbläschen (beliebig) verteilt in einer (oder mehreren) flüssigen Phase(n) vorliegen, wobei die Zubereitungen makroskopisch nicht notwendigerweise das Aussehen eines Schaumes haben müssen. Erfindungsgemäße kosmetische oder dermatologische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Nach dem Stand der Technik ließen sich selbstschäumende, schaumförmige, nachschäumende oder schäumbare kosmetische Emulsionen ohne Verwendung besonderer Tenside nicht formulieren bzw. technisch herstellen. Dieses galt insbesondere für Systeme, die auf klassischen Emulgatoren basieren. Nach dem Stand der Technik entwickelten derartige Systeme unter Zusatz von Treibgas ausschließlich wäßrig-feuchte Schäume, die nach Applikation schnell brachen.

Durch die Erfindung wird erstmalig ein gehaltvoller, kompakter Cremeschaum zugänglich, der sich über eine lange Lagerdauer sowie durch eine außerordentlich hohe Stabilität und ein kompaktes Erscheinungsbild auszeichnet.

Durch die Verwendung der erfindungsgemäßen Copolymere wird der Eintrag von Gasen unterstützt sowie über eine längere Lagerdauer auch bei höheren Temperaturen (z. B. 40 °C) ein stabilisierender sowie deutlich schaumsteigernder Effekt erzielt. Es war dabei insbesondere erstaunlich, daß auf die Verwendung besonderer Tenside verzichtet werden kann. Der Eintrag von Gasen ist gegenüber dem Stand der Technik überraschenderweise außerordentlich erhöht. So kann beispielsweise eine Schaumverstärkung mit bis zu 100%ig erhöhtem Gasvolumen erzielt werden, ohne nach dem Stand der Technik übliche Schäummittel wie Tenside zu verwenden.

Hierdurch ist es erstmals möglich, Rezepturen mit einer herausragenden, neuartigen kosmetischen Wirkleistung und mit außerordentlich hohem Gasvolumen (Luft und/oder andere Gase wie Sauerstoff, Kohlendioxid, Stickstoff, Helium, Argon u.a.) über lange Lagerdauer bei hohen Temperaturen stabil zu generieren. Gleichzeitig zeichnen sich die erfindungsgemäßen Zubereitungen durch eine überdurchschnittlich. gute Hautpflege sowie sehr gute sensorische Eigenschaften aus.

Gegenstand der Erfindung ist daher ferner
die Verwendung eines oder mehrerer nicht-ionischer polymerer Verdicker gewählt aus der Gruppe der Copolymere aus
(a) Polyethylenglykol der Formel HO-CH₂(CH₂-O-CH₂)ₙ-CH₂-OH, worin n eine ganze Zahl von 100 bis 250 bedeutet,
(b) Polyoxyethylenglykolalkylether der Formel HO(CH₂CH₂O)ₓR¹, worin x eine ganze Zahl von 1 bis 100 und R¹ einen verzweigten oder unverzweigten, gesättigten oder ungesättigten Alkylrest mit 4 bis 40 Kohlenstoffatomen darstellen, und optional
(c) Tetramethoxymethylglycoluril
zur Schaumverstärkung selbstschäumender, schaumförmiger, nachschäumender oder schäumbarer kosmetischer und dermatologischer Zubereitungen.

Unter "Schaumverstärkung" ist im Sinne der vorliegenden Erfindung zu verstehen, daß der Eintrag von Gasen in die erfindungsgemäßen Schäume gegenüber dem Eintrag in ansonsten gleiche Zubereitungen, welche keine erfindungsgemäßen Copolymere enthalten, außerordentlich erhöht ist. Die erfindungsgemäßen Schäume können dementsprechend ein deutlich höheres Gasvolumen aufnehmen als Zubereitungen, welche keine erfindungsgemäßen Copolymere enthalten.

Mit "Schaumverstärkung" ist darüberhinaus gemeint, daß die Stabilität der aufgeschäumten Zubereitungen (die "Schaumstabilität") gegenüber ansonsten gleichen Zubereitungen, welche keine erfindungsgemäßen Copolymere enthalten, deutlich verbessert wird, d. h. durch die erfindungsgemäße Verwendung wird ein Brechen der Schäume zeitlich verzögert.

Ferner ist unter "Schaumverstärkung" im Sinne der vorliegenden Erfindung zu verstehen, daß auch die kosmetischen Eigenschaften der erfindungsgemäßen Schäume im Vergleich zu Zubereitungen, welche keine erfindungsgemäßen Copolymere enthalten, deutlich verbessert werden: So erhält man durch die erfindungsgemäße Verwendung reichhaltige, feste Schäume ("Schaum-Cremes"), welche trotz ihrer Kompaktheit und Reichhaltigkeit leicht verteilbar sind und schnell einziehen.

Die erfindungsgemäßen Zubereitungen stellen in jeglicher Hinsicht überaus befriedigende Präparate dar. Es war insbesondere überraschend, daß die erfindungsgemäßen schaumförmigen Zubereitungen - auch bei einem ungewöhnlich hohen Gasvolumen - außerordentlich stabil sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische Eigenschaften, wie beispielsweise die Verteilbarkeit auf der Haut oder das Einzugsvermögen in die Haut, und zeichnen sich darüberhinaus durch eine überdurchschnittlich gute Hautpflege aus.

Vorteilhafte erfindungsgemäße Copolymere zeichnen sich durch die folgende Strukturformel (I) aus 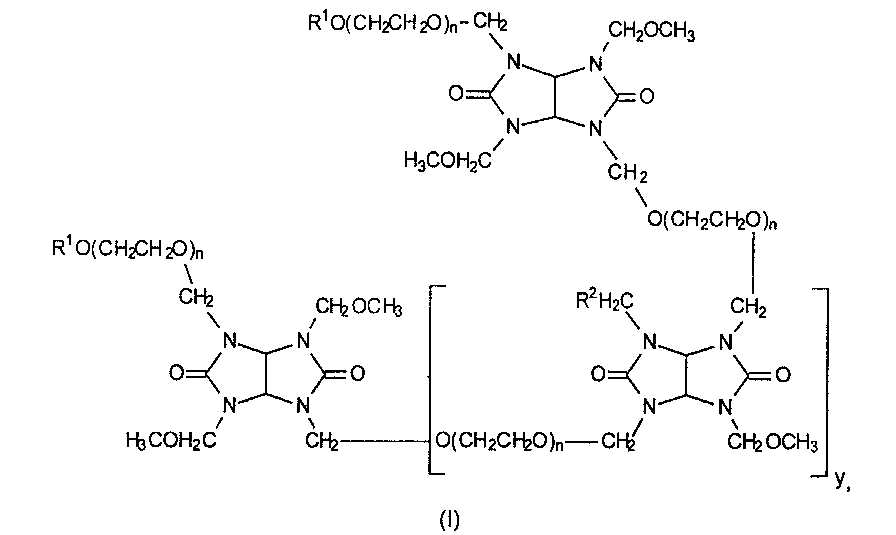 worin
- R¹ einen verzweigten oder unverzweigten, gesättigten oder ungesättigten Alkylrest mit 4 bis 40 Kohlenstoffatomen,
- R² = -OCH₃ oder -O(CH₂CH₂O)ₓR¹,
- x eine ganze Zahl von 1 bis 100,
- n eine ganze Zahl von 100 bis 250 und
- y durchschnittlich 2 oder 3
bedeuten.

Besonders vorteilhafte erfindungsgemäße Copolymere sind solche, für die n eine ganze Zahl von 150 bis 200 ist. Es ist insbesondere vorteilhaft im Sinne der vorliegenden Erfindung, wenn darüberhinaus R¹ einen verzweigten oder unverzweigten, gesättigten oder ungesättigten Alkylrest-mit 8 bis 12 Kohlenstoffatomen darstellt.

Es ist ferner vorteilhaft im Sinne der vorliegenden Erfindung, wenn die mittlere Molmasse der Copolymere zwischen 30.000 und 50.000 liegt.

Erfindungsgemäß ganz besonders vorteilhaft sind PEG-180/Octoxynol-40/Tetramethoxymethylglycoluril Copolymere mit R² = -O(CH₂CH₂O)₄₀C₈H₁₇ und n = 180.

Erfindungsgemäß ferner besonders vorteilhaft sind PEG-180/Laureth-50/Tetramethoxymethylglycoluril Copolymere mit R² = -O(CH₂CH₂O)₅₀C₁₂H₂₅ und n = 180.

Erfindungsgemäß ferner besonders vorteilhaft ist der Polyether-1.

Es ist vorteilhaft im Sinne der vorliegenden Erfindung, die Gesamtmenge der Copolymere aus dem Bereich von 0,01 bis 5 Gew.-%, vorteilhaft von 0,1 bis 1 Gew.-%, jeweils bezogen auf das Gesamtgewicht der Formulierung, zu wählen.

Besonders vorteilhafte Zubereitungen im Sinne der vorliegenden Erfindung enthalten ein Emulgatorsystem, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

Besonders bevorzugt im Sinne der vorliegenden Erfindung ist es, wenn die Gasphase der Zubereitungen Kohlendioxid enthält bzw. ganz aus Kohlendioxid besteht. Es ist insbesondere vorteilhaft, wenn Kohlendioxid einen oder den Wirkstoff in den erfindungsgemäßen Zubereitungen darstellt.

Erfindungsgemäße Zusammensetzungen entwickeln sich bereits während ihrer Herstellung - beispielsweise während des Rührens oder bei der Homogenisierung - zu feinblasigen Schäumen. Erfindungsgemäß sind feinblasige, reichhaltige Schäume von hervorragender kosmetischer Eleganz erhältlich. Weiterhin sind erfindungsgemäß besonders gut hautverträgliche Zubereitungen erhältlich, wobei wertvolle Inhaltsstoffe besonders gut auf der Haut verteilt werden können.

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureestern des Sorbitans.

Die Gesamtmenge der weiteren Emulgatoren wird erfindungsgemäß vorteilhaft kleiner als 5 Gew.-%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte Zubereitungen im Sinne der vorliegenden Erfindung sind frei von Mono- oder Diglycerylfettsäureestern. Insbesondere bevorzugt sind erfindungsgemäße Zubereitungen, welche kein Glycerylstearat, Glycerylisostearat, Glyceryldiisostearat, Glyceryloleat, Glycerylpalmitat, Glycerylmyristat, Glyceryllanolat und/oder Glyceryllaurat enthalten.

Die Ölphase der erfindungsgemäßen Zubereitungen wird vorteilhaft gewählt aus der Gruppe der unpolaren Lipide mit einer Polarität ≥ 30 mN/m und der cyclischen oder linearen Silikonöle. Besonders vorteilhafte unpolare Lipide im Sinne der vorliegenden Erfindung sind die im folgenden aufgelisteten.

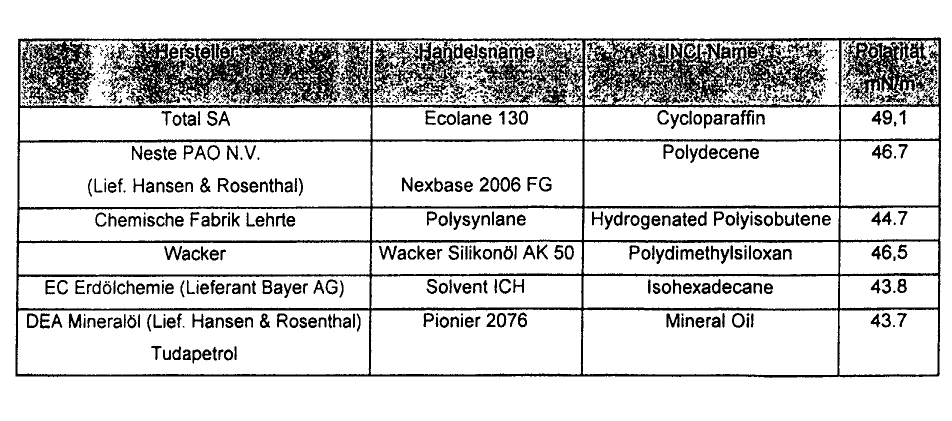

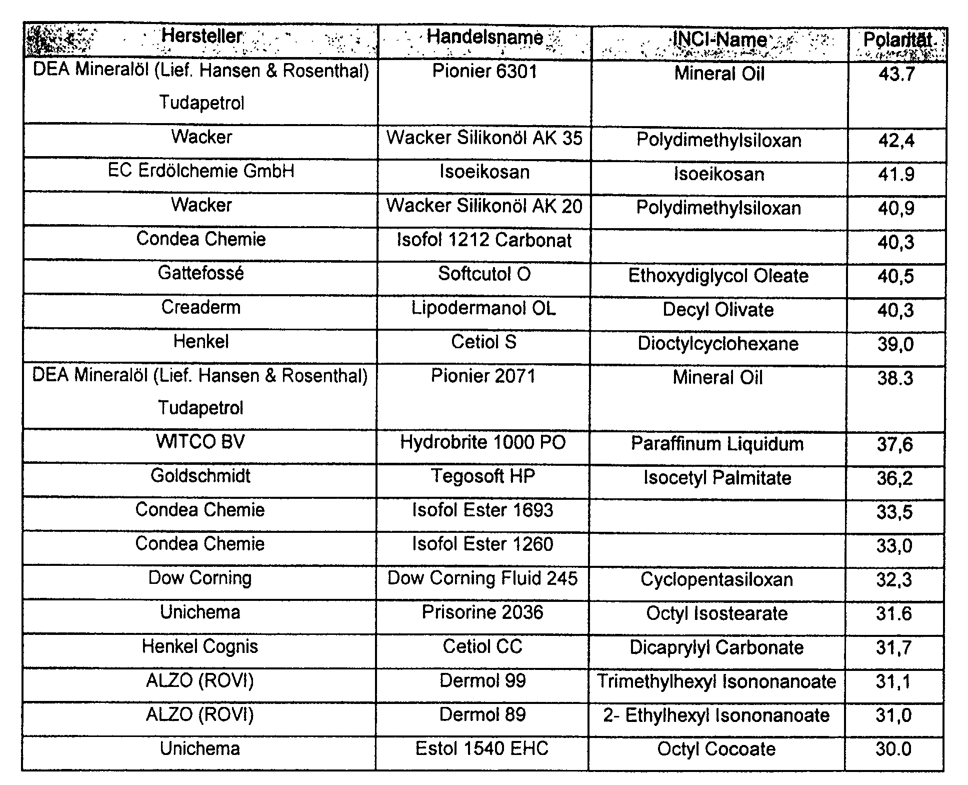

Von den Kohlenwasserstoffen sind insbesondere Paraffinöl sowie weitere hydrierte Polyolefine wie hydriertes Polyisobutene, Squalan und Squalen vorteilhaft im Sinne der vorliegenden Erfindung zu verwenden.

Die Gehalt der Lipidphase wird vorteilhaft kleiner als 50 Gew.-% gewählt, bevorzugt zwischen 2,5 und 30 Gew.-%, insbesondere bevorzugt zwischen 5 und 15 Gew.-%, jeweils bezogen auf das Gesamtgewicht der Zubereitung. Es ist gegebenenfalls ferner vorteilhaft, wenngleich nicht zwingend, wenn die Lipidphase bis zu 40 Gew.-% - bezogen auf das Gesamtgewicht der Lipidphase - an polaren Lipiden (mit einer Polarität ≤ 20 mN/m) und/oder mittelpolaren Lipiden (mit einer Polarität von 20 bis 30 mN/m) enthält.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

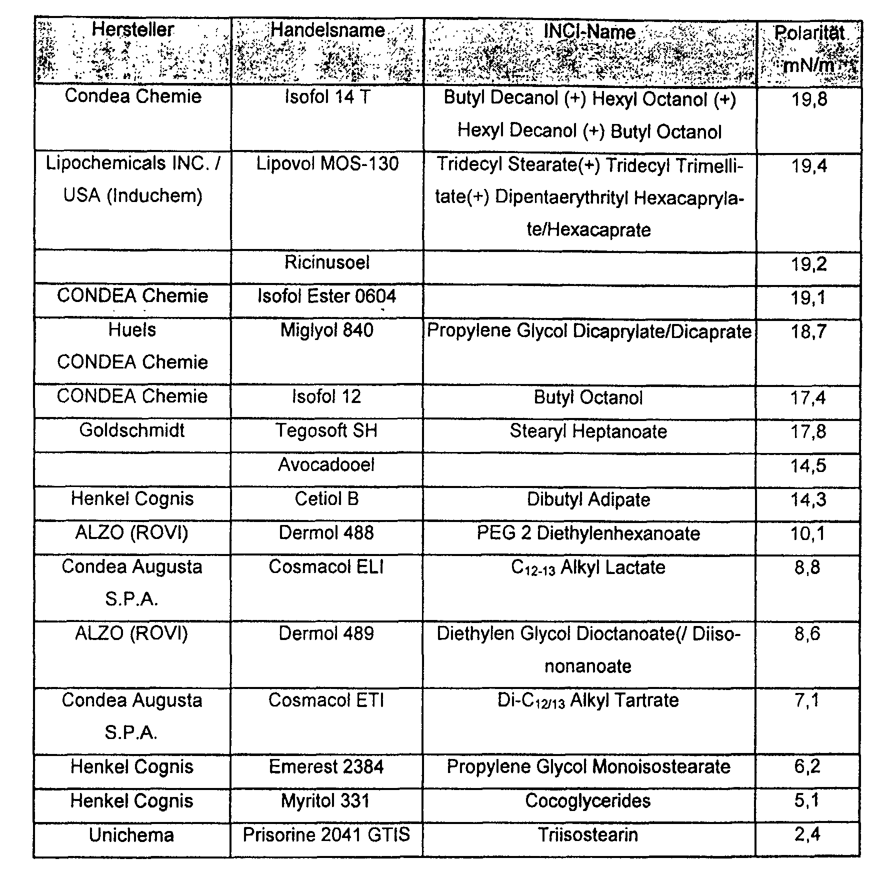

Besonders vorteilhafte mittelpolare Lipide im Sinne der vorliegenden Erfindung sind die im folgenden aufgelisteten.

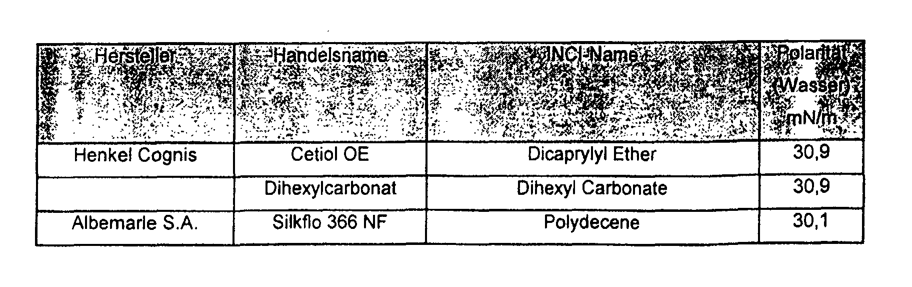

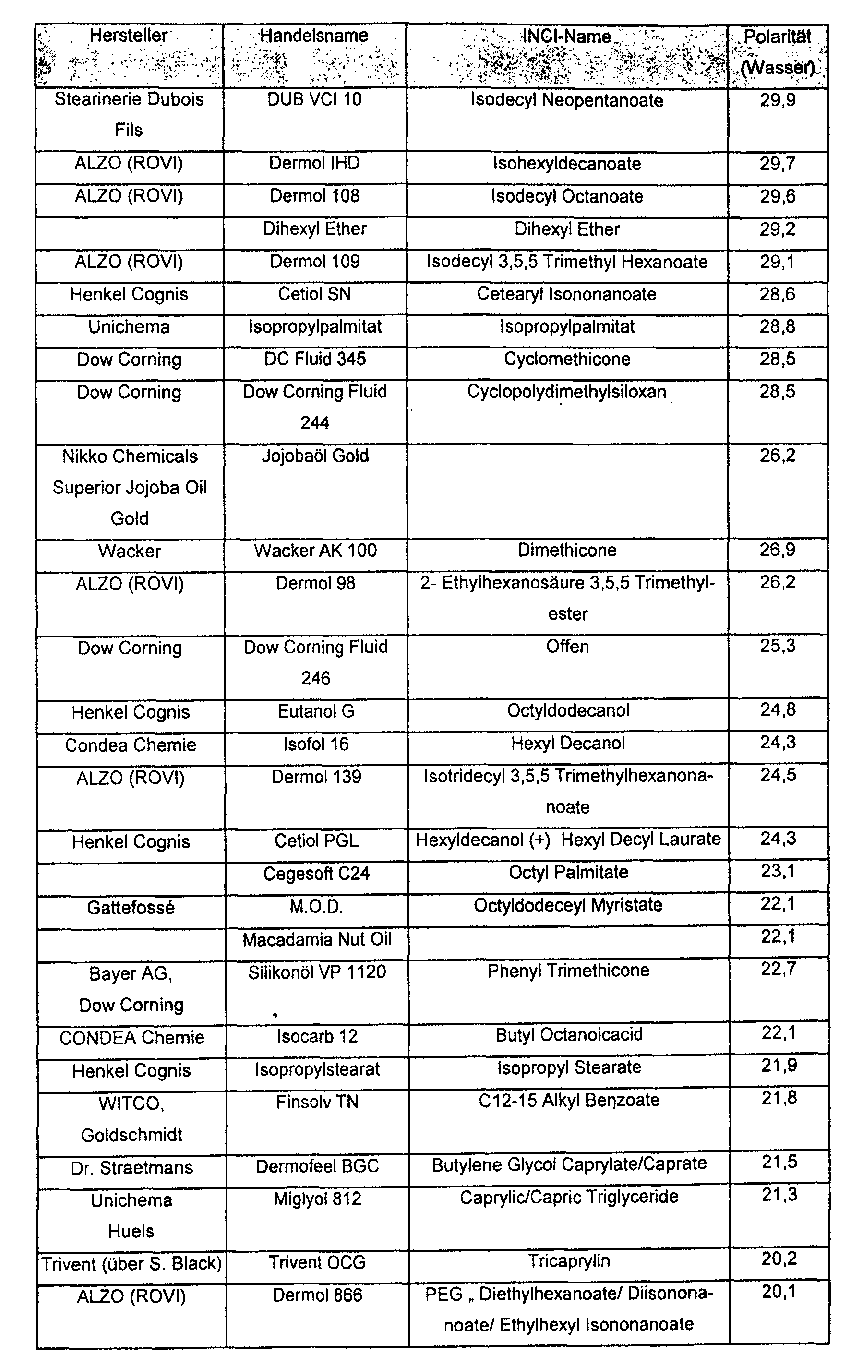

Die erfindungsgemäßen kosmetischen und/oder dermatologischen Zubereitungen können ferner vorteilhaft anorganische partikuläre hydrophobe und/oder hydrophobisierte und/oder ölabsorbierende Festkörpersubstanzen und/oder anorganische Gelbildner enthalten.

Vorteilhafte anorganische partikulären hydrophoben und/oder hydrophobisierten und/oder ölabsorbierenden Festkörpersubstanzen können beispielsweise gewählt werden aus der Gruppe
- der anorganischen Füllstoffe (wie Talkum, Kaolin, Zeolithe, Bornitrid),
- der anorganischen Pigmente auf Basis von Metalloxiden und/oder anderen in Wasser schwerlöslichen bzw. unlöslichen Metallverbindungen (insbesondere Oxide des Titans, Zinks, Eisens, Mangans, Aluminium, Cers),
- der anorganischen Pigmente auf Basis von Silicumoxiden (wie insbesondere die Typen Aerosil-200, Aerosil 200 V),
- der Silikat-Derivate (wie Natrium Silicoaluminate oder Fluoro Magnesium Silikate (Submica-Typen), Calcium Aluminium Borsilikate). Bevorzugt ist hierbei insbesondere Silica Dimethyl Silylate (Aerosil® R972).

Aerosile [(fumed Silica) = durch thermische Zersetzung von EthylSilikat gewonnenes Siliciumdioxid)] sind hochdisperse Kieselsäuren mit häufig irregulärer Form, deren spezifische Oberfläche in der Regel sehr groß ist (200 - 400 m²/ g) und abhängig vom Herstellverfahren gesteuert werden kann.

Erfindungsgemäß besonders vorteilhaft zu verwendende Aerosile sind beispielsweise erhältlich unter den Handelsnamen: Aerosil® 130 (Degussa Hüls) Aerosil® 200 (Degussa Hüls) Aerosil 255 (Degussa Hüls) Aerosil® 300 (Degussa Hüls) Aerosil® 380 (Degussa Hüls) B-6C (Suzuki Yushi) CAB-O-SIL Fumed Silica (Cabot) CAB-O- SIL EH-5 (Cabot) CAB-O-SIL HS-5 (Cabot) CAB-O-SIL LM-130 (Cabot) CAB-O-SIL MS-55 (Cabot) CAB-O-SIL M-5 (Cabot) E-6C (Suzuki Yushi) Fossil Flour MBK (MBK) MSS-500 (Kobo) Neosil CT 11 (Crosfield Co.) Ronasphere (Rona/EM Industries) Silica, Anhydrous 31 (Whittaker, Clark & Daniels) Silica, Crystalline 216 (Whittaker, Clark & Daniels) Silotrat-1 (Vevy) Sorbosil AC33 (Crosfield Co.) Sorbosil AC 35 (Crosfield Co.) Sorbosil AC 37 (Crosfield Co.) Sorbosil AC 39 (Crosfield Co.) Sorbosil AC77 (Crosfield Co.) Sorbosil TC 15 (Crosfield Co.) Spherica (Ikeda) Spheriglass (Potters-Ballotini) Spheron L-1500 (Presperse) Spheron N-2000 (Presperse) Spheron P-1500 (Presperse) Wacker HDK H 30 (Wacker-Chemie) Wacker HDK N 20 (Wacker-Chemie) Wacker HDK P 100 H (Wacker Silicones) Wacker HDK N 20P (Wacker-Chemie) Wacker HDK N 25P (Wacker-Chemie) Wacker HDK S 13 (Wacker-Chemie) Wacker HDK T 30 (Wacker-Chemie) Wacker HDK V 15 (Wacker-Chemie) Wacker HDK V 15 P (Wacker-Chemie) Zelec Sil (DuPont).

Weiterhin ist vorteilhaft, solche SiO₂-Pigmente zu verwenden, bei welchen die freien OH Gruppen an der Teilchenoberfläche (ganz oder teilweise) organisch modifiziert worden sind. Man erhält z. B. durch die Addition von Dimethylsilyl-Gruppen Silica Dimethyl Silylate (z. B. Aerosil® R972 (Degussa Hüls) Aerosil® R974 (Degussa Hüls) CAB-O-SIL TS-610 (Cabot) CAB-O-SIL TS-720 (Cabot) Wacker HDK H15 (Wacker-Chemie) Wacker HDK H18 (Wacker-Chemie) Wacker HDK H20 (Wacker-Chemie)). Durch die Addition von Trimethylsily-Gruppen erhält man Silica Silylate (z. B. Aerosil R 812 (Degussa Hüls) CAB-O-SIL TS-530 (Cabot) Sipernat D 17 (Degussa Hüls) Wacker HDK H2000 (Wacker-Chemie)).

Sehr vorteilhafte anorganische Gelbildner können beispielsweise gewählt werden aus der Gruppe der modifizierten oder unmodifizierten, natürlich vorkommenden oder synthetischen Schichtsilikate. Es ist zwar durchaus günstig, reine Komponenten einzusetzen, die erfindungsgemäßen Zubereitungen können jedoch auch in vorteilhafter Weise Gemische verschiedener modifizierter und/oder unmodifizierter Schichtsilikate enthalten.

Unter Schichtsilikaten, welche auch Phyllosilikate genannt werden, sind im Rahmen dieser Anmeldung Silikate und Alumosilikate zu verstehen, in welchen die Silikat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O-Valenz wird durch Kationen abgesättigt. Zwischen den einzelnen Schichten bestehen schwächere elektrostatische Wechselwirkungen, z. B. Wasserstoffbrückenbindungen. Das Schichtgefüge indessen ist weitgehend durch starke, kovalente Bindungen geprägt.

Die Stöchiometrie der Blattsilikate ist
(Si₂O₅²⁻) für reine Silikatstrukturen und
(AlₘSi²⁻ₘO₅(^{2+m})⁻) für Alumosilikate.
m ist eine Zahl größer als Null und kleiner als 2.

Liegen keine reinen Silikate, sondern Alumosilikate vor, ist dem Umstand Rechnung zu tragen, daß jede durch Al³⁺ ersetzte Si⁴⁺ - Gruppe ein weiteres einfach geladenes Kation zur Ladungsneutralisierung erfordert.

Die Ladungsbilanz wird bevorzugt durch H⁺, Alkali- oder Erdalkalimetallionen ausgeglichen. Auch Aluminium als Gegenion ist bekannt und vorteilhaft. Im Gegensatz zu den Alumosilikaten werden diese Verbindungen Aluminiumsilikate genannt. Auch "Aluminiumalumosilikate", in welchen Aluminium sowohl im Silikatnetz, als auch als Gegenion vorliegt, sind bekannt und für die vorliegende Erfindung gegebenenfalls von Vorteil.

Schichtsilikate sind in der Literatur gut dokumentiert, z. B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H.Remy, 12. Aufl., Akademische Verlagsgesellschaft, Leipzig 1965, passim. Die Schichtenstruktur von Montmorillonit ist Römpps Chemie-Lexikon, Franckh'sche Verlagshandlung W. Keller & Co., Stuttgart, 8.Aufl., 1985, S. 2668 f., zu entnehmen.

Beispiele für Schichtsilikate sind:

| | |
|---|---|
| Montmorillonit | Na_{0,33}((Al_{1,67}Mg_{0,33})(OH)₂(S₁₄O₁₀)) |
| oft vereinfacht: | Al₂O₃*4SiO₂*H₂O*nH₂O bzw. Al₂[(OH)₂/Si₄O₁₀]·n H₂O |
| Kaolinit | Al₂(OH)₄(Si₂O₅) |
| Ilit | (K,H₃O)_{y}(Mg₃(OH)₂(Si_{4-y}Al_{y}O₁₀)) |
| und | (K,H₃O)_{y}(Al₂(OH)₂(Si_{4-y}Al_{y}O₁₀)) mit y = 0,7 - 0,9 |
| Beidellit | (Ca,Na)_{0,3}(Al₂(OH)₂(Al_{0,5}Si_{3,5}O₁₀)) |
| Nontronit | Na_{0,33}(Fe₂(OH)₂(Al_{0,33}S_{i3,67}O₁₀)) |
| Saponit | (Ca,Na)_{0,33}((Mg,Fe)₃(OH)₂(Al_{0,33}Si_{3,67}O₁₀)) |
| Hectorit | Na_{0,33}((Mg,Li)₃(OH,F)₂(Si₄O₁₀)) |

Montmorillonit stellt das Hauptmineral der natürlich vorkommenden Bentonite dar.

Sehr vorteilhafte anorganische Gelbildner im Sinne der vorliegenden Erfindung sind Aluminiumsilikate wie die Montmorillonite (Bentonite, Hectorite sowie deren Derivate wie Quaternium-18 Bentonit, Quaternium-18 Hectorite, Stearalkonium Bentonite bzw. Stearalkonium Hectorite) oder aber Magnesium-Aluminium-Silikate (Veegum®-Typen) sowie Natrium-Magnesium-Silikate (Laponite®-Typen).

Montmorillonite stellen zu den dioktaedrischen Smektiten gehörende Tonmineralien dar und sind in Wasser quellende, aber nicht plastisch werdende Massen. Die Schichtpakete in der Dreischicht-Struktur der Montmorillonite können durch reversible Einlagerung von Wasser (in der 2-7fachen Menge) u. a. Substanzen wie z. B. Alkoholen, Glykolen, Pyridin, α-Picolin, Ammonium-Verbindungen, Hydroxy-AluminoSilikat-lonen usw. aufquellen.

Die oben angegebene chemische Formel ist nur angenähert; da Montmorillonit ein großes lonenaustausch-Vermögen besitzt, kann Al gegen Mg, Fe²⁺, Fe³⁺, Zn, Pb, Cr, auch Cu und andere ausgetauscht werden. Die daraus resultierende negative Ladung der Oktaeder-Schichten wird durch Kationen, insbesondere Na⁺ (Natrium-Montmorillonit) und Ca²⁺ (der Calcium-Montmorillonit ist nur sehr wenig quellfähig) in Zwischenschicht-Positionen ausgeglichen.

Im Sinne der vorliegenden Erfindung vorteilhafte synthetische Magnesiumsilikate bzw. Bentonite werden beispielsweise von Süd-Chemie unter der Handelsbezeichung Optigel® vertrieben.

Ein im Sinne der vorliegenden Erfindung vorteilhaftes Aluminiumsilikat wird beispielsweise von der R. T. Vanderbilt Comp., Inc., unter der Handelsbezeichnung Veegum® vertrieben. Die verschiedenen Veegum®-Typen, welche alle erfindungsgemäß vorteilhaft sind, zeichnen sich durch folgende Zusammensetzungen aus

| | (regular grade) | HV | K | HS | S-728 |
|---|---|---|---|---|---|
| SiO₂ | 55,5 | 56,9 | 64,7 | 69,0 | 65,3 |
| MgO | 13,0 | 13,0 | 5,4 | 2,9 | 3,3 |
| Al₂O₃ | 8,9 | 10,3 | 14,8 | 14,7 | 17,0 |
| Fe₂O₃ | 1,0 | 0,8 | 1,5 | 1,8 | 0,7 |
| CaO | 2,0 | 2,0 | 1,1 | 1,3 | 1,3 |
| Na₂O | 2,1 | 2,8 | 2,2 | 2,2 | 3,8 |
| K₂O | 1,3 | 1,3 | 1,9 | 0,4 | 0,2 |
| Veraschungsverlust | 11,1 | 12,6 | 7,6 | 5,5 | 7,5 |

Diese Produkte quellen in Wasser unter Bildung viskoser Gele, welche alkalisch reagieren. Durch Organophilierung von Montmorillonit bzw. Bentoniten (Austausch der Zwischenschicht-Kationen gegen quaternäre Alkylammonium-lonen) entstehen Produkte (Bentone), die bevorzugt zur Dispergierung in organischen Lösemitteln und Ölen, Fetten, Salben, Farben, Lacken und in Waschmitteln eingesetzt werden.

Bentone® ist eine Handelsbezeichnung für verschiedene neutrale und chemisch inerte Geliermittel, die aus langkettigen, organischen Ammoniumsalzen und speziellen Montmorillonit-Sorten aufgebaut sind.

Folgende Bentone® -Typen werden beispielsweise von der Gesellschaft Kronos Titan vertrieben und sind vorteilhaft im Sinne der vorliegenden Erfindung einzusetzen: Bentone® 27, ein organisch modifiziertes Montmorillonit, Bentone® 34 (Dimethyldioctylammoniumbentonit), das nach US 2,531,427 hergestellt wird und wegen seiner lipophilen Gruppen besser im lipophilen Medium als in Wasser quillt, Bentone® 38, ein organisch modifiziertes Montmorillonit, ein cremefarbenes bis weißes Pulver, Bentone® LT, ein gereinigtes Tonmineral, Bentone® Gel MIO, ein organisch modifiziertes Montmorillonit, das in Mineralöl (SUS-71) feinst suspendiert angeboten wird (10 % Bentonit, 86,7 % Mineralöl und 3,3 % Netzmittel), Bentone® Gel IPM, ein organisch modifiziertes Bentonit, das in Isopropylmyristat suspendiert ist (10 % Bentonit, 86,7 % Isopropylmyristat, 3,3 % Netzmittel), Bentone® Gel CAO, ein organisch modifiziertes Montmorillonit, das in Ricinusöl aufgenommen ist (10 % Bentonit, 86,7 % Ricinusöl und 3,3 % Netzmittel), Bentone® Gel Lantrol, ein organisch modifiziertes Montmorillonit, das in Pastenform zur Weiterverarbeitung, insbesondere zur Herstellung kosmetischer Mittel bestimmt ist; 10 % Bentonit, 64,9 Lantrol (Wollwachsöl), 22,0 Isopropylmyristat, 3,0 Netzmittel und 0,1 p-Hydroxybenzoesäurepropylester, Bentone® Gel Lan I, eine 10 %ige Bentone® 27-Paste in einer Mischung aus Wollwachs USP und Isopropylpalmitat, Bentone® Gel Lan II, eine Bentonit-Paste in reinem, flüssigem Wollwachs, Bentone® Gel NV, eine 15 %ige Bentone® 27-Paste in Dibutylphthalat, Bentone® Gel OMS, eine Bentonit-Paste in Shellsol T. Bentone® Gel OMS 25, eine Bentonit Paste in Isoparaffinischen Kohlenwasserstoffen (Idopar® H), Bentone® Gel IPP, eine Bentonit-Paste in Isopropylpalmitat.

Alle Bentone-Typen sind vorteilhaft im Sinne der vorliegenden Erfindung zu verwenden.

Vorteilhaft können Zubereitungen im Sinne der vorliegenden Erfindung ferner ein oder mehrere Hydrokolloide aus einer oder mehreren der folgenden Gruppen enthalten:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane.

Die erfindungsgemäßen kosmetischen und/oder dermatologischen Zubereitungen können wie üblich zusammengesetzt sein. Besonders vorteilhaft im Sinne der vorliegenden Erfindung sind Zubereitungen zur Pflege der Haut: sie können dem kosmetischen und/oder dermatologischen Lichtschutz, ferner zur Behandlung der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische oder topische dermatologische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Ebenso wie Emulsionen von flüssiger und fester Konsistenz als kosmetische Reinigungslotionen bzw. Reinigungscremes Verwendung finden, können auch die erfindungsgemäßen Zubereitungen "Reinigungsschäume" darstellen, welche beispielsweise zum Entfernen von Schminken und/oder Make-up oder als milder Waschschaum - ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die kosmetischen oder dermatologischen Mittel gemäß der Erfindung können beispielsweise aus Aerosolbehältern entnommen und dabei aufgeschäumt werden. Erfindungsgemäße Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Deratige Behälter können mit Ventilen sehr unterschiedlicher Bauart ausgestattet sein, die die Entnahme des Inhalts als Schaum ermöglichen.

Ferner vorteilhaft können die Zubereitungen gemäß der vorliegenden Erfindung aus Treibgas-freien, mechanisch zu bedienenden Pumpzerstäubern (Pumpspendern) entnommen werden. Insbesondere vorteilhaft im Sinne der vorliegenden Erfindung sind Pumpsysteme, welche ohne Druckgas, aber mit einem Filter, der spezielle Verwirbelungen bewirkt, arbeiten.

Die kosmetischen oder dermatologischen Zubereitungen gemäß der Erfindung können ferner vorzugsweise beispielsweise aus Zweikammeraerosolbehältern entnommen und auf die Haut aufgetragen werden. Erfindungsgemäß vorteilhafte Packmittel sind Behältnisse, in denen sich eine Kammer mit einer Füllung aus den flüssigen bzw. breiartigen Zubereitungen unter dem Druck eines in einer zweiten Kammer befindlichen stehenden Primärtreibmittels befindet. Derartige Behälter können mit Ventilen sehr unterschiedlicher Bauart ausgestattet sein, die die Entnahme des Inhalts der ersten Kammer als Emulsion oder Gel in jeder Lage - auch mit dem Ventil nach unten - ermöglichen.
Eine vorteilhafte Ausführungsform sind BiCan®-Aerosolbehälter, bei denen das Produkt in einem flexiblen Beutel aus Metall oder Kunststoff innerhalb der Dose eingeschlossen ist.

Erfindungsgemäße nachschäumende Zusammensetzungen stellen ungeschäumt, also unmittelbar nach dem Austreten aus einem Aerosolbehälter, Zwei- oder Mehrphasensysteme - in der Regel Emulsionen - dar. Sie können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.

Es hat sich darüber hinaus in überraschender Weise herausgestellt, daß bei der Verwendung von (Sekundär-) Treibmitteln, besonders vorteilhaft von in der gegebenenfalls vorhandenen Ölphase löslichen Treibmitteln, also beispielsweise üblichen Propan-Butan-Gemischen, die erfindungsgemäßen Zubereitungen nicht einfach als Aerosoltröpfchen versprüht werden, sondern sich zu feinblasigen, reichhaltigen Schäumen entwickeln, sobald solche mit solchen (Sekundär-) Treibmitteln beladenen Systeme Druckentspannung erfahren.

Bei Verwendung von Kohlenwasserstoffen oder deren Gemischen mit 4 oder 5 Kohlenstoffatomen, insbesondere Isobutan, n-Pentan und Isopentan, als (Sekundär-) Treibmittel kann man das selbständige Aufschäumen nach dem Austritt aus der Druckverpackung zeitlich verzögern.

Durch das Verdampfen des Sekundärtreibmittels im applizierten Kosmetikprodukt wird der Haut ferner Wärme entzogen und ein angenehmer Kühleffekt erlangt. Solche nachschäumenden Zubereitungen werden daher ebenfalls als vorteilhafte Verkörperungen der vorliegenden Erfindung mit eigenständiger erfinderischer Tätigkeit angesehen.

Als Druckgasbehälter kommen im Sinne der vorliegenden Erfindung vor allem zylindrische Gefäße aus Metall (Aluminium, Weißblech, Inhalt <1000 mL), geschütztem bzw. nicht-splitterndem Glas oder Kunststoff (Inhalt < 220 mL) bzw. splitterndem Glas oder Kunststoff (Inhalt < 150 mL) in Frage, bei deren Auswahl Druck- und Bruchfestigkeit, Korrosionsbeständigkeit, leichte Füllbarkeit, ggf. Sterilisierbarkeit usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 12 bar und das maximale Füllvolumen bei dieser Temperatur ca. 90 % des Gesamtvolumens. Für Glasund Kunststoffdosen gelten niedrigere, von der Behältergröße und dem Treibmittel (ob verflüssigtes, verdichtetes oder gelöstes Gas) abhängige Werte für den Betriebsdruck.

Besonders vorteilhaft im Sinne der vorliegenden Erfindung sind Dosen aus Weißblech, Aluminium und Glas. Aus Korrosionsschutzgründen können Metalldosen innen lackiert sein (silber- oder goldlackiert), wozu alle handelsüblichen Innenschutzlacke geeignet sind. Bevorzugt im Sinne der vorliegenden Erfindung sind Polyester-, Epoxyphenol- sowie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt. Werden Kunststoffe als Sprüh-Behältermaterial verwendet, so sollten diese Chemikalien- und Sterilisationstemperatur-beständig, gasdicht, schlagfest und gegen Innendrücke über 12 bar stabil sein. Prinzipiell für Sprüh-Behälter-Zwecke geeignet sind Polyacetale und Polyamide.

Der innere Aufbau der Sprüh-Dosen sowie die Ventilkonstruktion sind je nach Verwendungs-Zweck und der physikalischen Beschaffenheit des Inhalts - z. B. ob als Zwei- oder als Dreiphasensystem - sehr variantenreich und können vom Fachmann durch einfaches Ausprobieren ohnen erfinderisches Zutun ermittelt werden. Für geeignete Ausführungsformen sei auf das "Aerosol Technologie Handbuch der Aersosol-Verpackung" hingewiesen (*Wolfgang Tauscher*, *Melcher Verlag GmbH Heidelberg*/*München,* 1996).

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:
- Teller:: Weißblech: blank, gold- bzw. klarlackiert, folienkaschiert (PE, PP oder PET) Aluminium: blank, silber- oder goldlackiert, verschiedene Lackvarianten, Stoner-Mudge-Ausführung
- Dichtung:: natürliche bzw. synthetische Elastomere bzw. thermoplastische (Sleeve-Gaskets, folienkaschiert aus PE oder PP) Innen- und Aussendichtungen, z. B. aus Perbunan, Buna, Neopren, Butyl, CLB, LDPE, Viton, EPDM, Chlorbutyl, Brombutyl und/oder diversen Compounds
- Kegel:: PA, POM, Messing sowie diversen Sondermaterialen,
Standardbohrungen (z. B.: 0,25 bis 0,70 mm oder 2 x 0,45 bis 2 x 1,00 mm), verschiedene Schaftdurchmesser
- Feder:: Metall, besonders bevorzugt V2A, rostfreier Stahl;
Kunststoff und auch Elastomer
- Gehäuse:: Standard und Impact
VPH-Bohrungen, RPT-Bohrungen oder geschlitzt für Überkopf-Anwendungen Materialien: z. B. Polyacetal, PA, PE, POM und dergleichen mehr
- Steigrohr:: Kunststoff (Polymer Resin), z.B. PE, PP, PA oder Polycarbonat

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Als Treibmittel sind die üblichen "klassischen" leichtflüchtigen, verflüssigten Treibgase, wie beispielsweise Dimethylether (DME) und/oder lineare oder verzweigtkettige Kohlenwasserstoffe mit zwei bis fünf Kohlenstoffatomen (wie insbesondere Ethan, Propan, Butan, Isobutan und/oder Pentan) geeignet, die allein oder in Mischung miteinander eingesetzt werden können.

Auch Druckluft sowie weitere unter Druck befindliche Gase wie Luft, Sauerstoff, Stickstoff, Wasserstoff, Helium, Krypton, Xenon, Radon, Argon, Lachgas (N₂O) und Kohlendioxid (CO₂) sind vorteilhaft im Sinne der vorliegenden Erfindung als Treibgase (sowohl einzeln als in beliebigen Mischungen miteinander) zu verwenden.

Natürlich weiß der Fachmann, daß es weitere an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere halogenierte (mit Fluor, Clor, Brom, lod und/oder Astat substituierte) Kohlenwasserstoffe wie beispielsweise Fluorkohlenwasserstoffe und Fluorchlorkohlenwasserstoffe (FCKW).

Die genannten Gase können im Sinne der vorliegenden Erfindung jeweils einzeln oder in beliebigen Mischungen zueinander verwendet werden.

Vorteilhaft im Sinne der vorliegenden Erfindung wird der Volumenanteil an Treibgas aus dem Bereich von 0,1 bis 30 Vol.-%, bezogen auf das Gesamtvolumen aus Füllgut und Treibgas gewählt (entsprechend einem Volumenanteil von 70 bis 99,9 Vol.-% Füllgut).

Besonders bevorzugtes Treibgas im Sinne der vorliegenden Erfindung ist Kohlendioxid. Insbesondere vorteilhaft sind aus erfindungsgemäßen Zubereitungen erhältliche Schäume, welche Kohlendioxid als einen oder den Wirkstoff enthalten.

Besonders vorteilhafte, feincremige und reichhaltige Schäume sind erhältlich, wenn die erfindungsgemäßen Zubereitungen mit Hilfe von linearen oder verzweigtkettigen, halogenierten oder nicht-halogenierten Kohlenwasserstoffen aufgeschäumt werden. Ganz besonders vorteilhafte Schäume sind durch Aufschäumen der erfindungsgemäßen Zubereitungen mit Kohlendioxid, Sauerstoff, Druckluft, Helium, Krypton, Xenon, Radon, Argon und/oder Stickstoff (sowohl einzeln als in beliebigen Mischungen miteinander) erhältlich.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Farbstoffe, Pigmente, die eine färbende Wirkung haben, anfeuchtende und/oder feuchthaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Erstaunlicherweise können ausgewählte erfindungsgemäße Rezepturen auch eine Antifaltenwirkung aufweisen bzw. die Wirkung bekannter Antifaltenwirkstoffe erheblich steigern. Dementsprechend eignen sich Formulierungen im Sinne der vorliegenden Erfindung insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

In einer besonderen Ausführungsform betrifft die vorliegende Erfindung daher Produkte zur Pflege der auf natürliche Weise gealterten Haut, sowie zur Behandlung der Folgeschäden der Lichtalterung, insbesondere der oben aufgeführten Phänomene.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index, 3. Auflage, Society of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   - "Fischsilber" (Guanin/Hypoxanthin-Mischkristalle aus Fischschuppen) und
   - "Perlmutt" (vermahlene Muschelschalen)
2. Monokristalline Perlglanzpigmente wie z. B. Bismuthoxychlorid (BiOCI)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂/Cr₂O₃ | grün |
| | TiO₂ / Berliner Blau | tiefblau |
| | TiO2 / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz und/oder ein oder mehrere Silikonderivate als weitere Phase enthalten. Ölfreie Formulierungen im Sinne der vorliegenden Erfindung können vorteilhaft auch weitere lipophile Komponenten - wie beispielsweise lipophile Wirkstoffe - enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCl: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCl: Octyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-Filtersubstanzen können öllöslich oder wasserlöslich sein.
Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Die Liste der genannten UV-Filter, die im Sinne der vorliegenden Erfindung eingesetzt werden können, soll selbstverständlich nicht limitierend sein.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1 (schaumförmige O/W-Creme)**: | | |
|---|---|---|
| Emulsion I | Gew.-% | Vol.-% |
| Stearinsäure | 3,00 | |
| Cetylalkohol | 8,50 | |
| PEG-20-Stearat | 8,50 | |
| Talkum | 2,00 | |
| SiO₂ | 2,00 | |
| Polyacrylsäure | 0,20 | |
| Magnesiumaluminiumsilikat | 0,50 | |
| Paraffinöl | 5,00 | |
| Isohexadecan | 2,00 | |
| PEG-180/Laureth-50/TMMG Copolymer | 0,50 | |
| Glycerin | 5,00 | |
| Natriumhydroxid | q.s. | |
| Konservierung | q.s. | |
| Parfum | q.s. | |
| Wasser, demineralisiert | ad 100,00 | |
| pH-Wert eingestellt auf 6,5-7,5 | | |
| Emulsion I | | 70 |
| Stickstoff | | 30 |

Vordispergierung des anorganischen Gelbildners und Quellung des Hydrokolloides sowie des Polymers unter Rühren in der Wasserphase. Vereinigung der auf 75 °C aufgeheizten Fettphase mit der auf 70 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Begasung mit Stickstoff bei 0.7bar und Kühlung. Zugabe der Additive bei 30 °C (Parfüm, Wirkstoffe). Homogenisierung mittels einer Zahnkranzdispergiermaschine (Rotor-Stator-Prinzip) bei 27 °C.

| **Beispiel 2 (schaumförmige O/W-Lotion):** | | |
|---|---|---|
| Emulsion II | Gew.-% | Vol.-% |
| Stearinsäure | 2,00 | |
| Myristylalcohol | 1,50 | |
| Cetylstearylalcohol | 0,50 | |
| PEG-100-Stearat | 3,00 | |
| Talkum | 0,05 | |
| Hydroxyethylcellulose | 0,05 | |
| Magnesiumaluminiumsilikat | 0,20 | |
| Mineralöl | 5,00 | |
| Hydriertes Polyisobuten | 15,0 | |
| PEG-180/Laureth-50/TMMG Copolymer | 0,50 | |
| Glycerin | 3,00 | |
| Natriumhydroxid | q.s. | |
| Konservierung | q.s. | |
| Parfum | q.s. | |
| Wasser, demineralisiert | ad 100,00 | |
| pH-Wert eingestellt auf 5,0-6,5 | | |
| Emulsion II | | 50 |
| Gas (Kohlendioxid) | | 50 |

Vordispergierung des anorganischen Gelbildners und Quellung des Hydrokolloides sowie des Polymers unter Rühren in der Wasserphase. Vereinigung der auf 80 °C aufgeheizten Fettphase mit der auf 72 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Begasung mit Kohlendioxid bei 1.2 bar und Kühlung. Zugabe der Additive bei 30 °C (Parfüm). Homogenisierung mittels einer Zahnkranzdispergiermaschine (Rotor-Stator-Prinzip) bei 30 °C.

| **Beispiel 3 (schaumförmige O/W-Lotion):** | | |
|---|---|---|
| Emulsion III | Gew.-% | Vol.-% |
| Stearinsäure | 5,00 | |
| Cetylstearylalkohol | 5,50 | |
| PEG-30-Stearat | 1,00 | |
| Aluminium-Stärkeoctenylsuccinat | 3,00 | |
| Al₂O₃ | 0,50 | |
| Talkum | 0,50 | |
| Polyurethan | 0,10 | |
| Polyacrylmethacrylat | 0,10 | |
| Magnesiumsilikat | 0,10 | |
| Cellulosegummi | 0,10 | |
| PEG-180/Octoxynol-40/TMMG Copolymer | 0,25 | |
| Cyclomethicon | 3,00 | |
| Isoeikosan | 10,00 | |
| Polydecen | 10,00 | |
| Citronensäure | 0,10 | |
| Glycerin | 3,00 | |
| Parfüm, Konservierungsmittel, | q.s. | |
| Natriumhydroxid | q.s. | |
| Farbstoffe usw. | q.s. | |
| Wasser | ad 100,00 | |
| pH-Wert eingestellt auf 6,0-7,5 | | |
| Emulsion III | | 65 |
| Gas (Luft) | | 35 |

Vordispergierung des anorganischen Gelbildners und Quellung der Hydrokolloide sowie des Polymers unter Rühren in der Wasserphase. Vereinigung der auf 80 °C aufgeheizten Fettphase mit der auf 75 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren in einem offenen Kessel bis auf 30 °C. Zugabe der Additive bei 30 °C (Parfüm, Wirkstoffe). Homogenisierung mittels einer Zahnkranzdispergiermaschine (Rotor-Stator-Prinzip) bei 25 °C.

| **Beispiel 4 (schaumförmige O/W-Emulsions-Make-up):** | | |
|---|---|---|
| Emulsion IV | Gew.-% | Vol.-% |
| Palmitinsäure | 2,00 | |
| Cetylalkohol | 2,00 | |
| PEG-100-Stearat | 2,00 | |
| Polyacrylsäure | 0,10 | |
| Aluminium-Stärkeoctenylsuccinat | 0,05 | |
| Maniokstärke | 0,05 | |
| Zeolithe | 0,75 | |
| Kaolin | 4,50 | |
| Natrium-Magnesium-Silikat | 0,15 | |
| Polyether-1 | 1,00 | |
| Dimethicon | 0,50 | |
| Paraffinöl | 9,50 | |
| Dicaprylylether | 2,00 | |
| Glycerin | 3,00 | |
| Glimmer | 1,00 | |
| Eisenoxide | 1,00 | |
| Titandioxid | 4,50 | |
| Vitamin-A-Palmitat | 0,10 | |
| Hectorit | 0,10 | |
| Natriumhydroxid | q.s. | |
| Konservierung | q.s. | |
| Parfum | q.s. | |
| Wasser, demineralisiert | ad 100,00 | |
| pH-Wert eingestellt auf 6,0 -7,5 | | |
| Emulsion IV | | 37 |
| Gas (Sauerstoff) | | 63 |

Vordispergierung der anorganischen Gelbildner und Quellung des Hydrokolloids sowie des Polymers unter Rühren in der Wasserphase. Vereinigung der auf 78 °C aufgeheizten Fett- und Pigmentphase mit der auf 75 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren im Becomix unter Begasung mit Sauerstoff bei 1,3 bar unter Kühlung auf 30 °C. Zugabe des Aluminium-Stärkeoctenylsuccinates, der Maniokstärke des Parfüms und der Wirkstoffe bei 30 °C. Homogenisierung mittels einer Zahnkranzdispergiermaschine (Rotor-Stator-Prinzip) bei 25 °C.

| **Beispiel 5 (schaumförmige O/W-Creme):** | | |
|---|---|---|
| Emulsion V | Gew.-% | Vol.-% |
| Stearinsäure | 4,00 | |
| Cetylalkohol | 2,00 | |
| PEG-30-Stearat | 2,00 | |
| Sorbitanmonostearat | 1,50 | |
| Paraffinöl | 5,00 | |
| Cyclomethicon | 1,00 | |
| Vitamin-E-Acetat | 1,00 | |
| Retinylpalmitat | 0,20 | |
| Glycerin | 3,00 | |
| BHT | 0,02 | |
| Na₂H₂EDTA | 0,10 | |
| Polyurethan | 0,10 | |
| Carboxymethylcellolose | 0,05 | |
| Polyacrylsäure | 0,10 | |
| Quaternium-18-Hectorit | 0,20 | |
| Magnsiumaluminiumsilikate | 0,10 | |
| Siliziumdioxid | 0,05 | |
| Talkum | 1,00 | |
| Polyether-1 | 0,10 | |
| Parfüm, Konservierungsmittel, | q.s. | |
| Farbstoffe | q.s. | |
| Kaliumhydroxid | q.s. | |
| Wasser | ad 100,00 | |
| pH-Wert eingestellt auf 5,0-7,0 | | |
| Emulsion V | | 43 |
| Gas (Lachgas) | | 57 |

Vordispergierung der anorganischen Gelbildner und Quellung des Hydrokolloids sowie des Polymers unter Rühren in der Wasserphase. Vereinigung der auf 80 °C aufgeheizten Fettphase mit der auf 75 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren im Becomix unter Begasung mit Lachgas bei 0.7 bar unter Kühlung auf 30 °C. Zugabe der Additive bei 30 °C (Parfüm, Wirkstoffe). Homogenisierung mittels einer Zahnkranzdispergiermaschine (Rotor-Stator-Prinzip) bei 26 °C.

| **Beispiel 6 (schaumförmige O/W-Lotion):** | | |
|---|---|---|
| Emulsion VI | Gew.-% | Vol.-% |
| Stearinsäure | 4,00 | |
| Cetylstearylalkohol | 1,00 | |
| PEG-100-Stearat | 1,00 | |
| PEG-100-Stearat | 1,00 | |
| Distärkephosphat | 0,50 | |
| Paraffinöl | 6,50 | |
| Dimethicon | 0,50 | |
| Vitamin-E-Acetat | 2,00 | |
| Glycerin | 3,00 | |
| Carboxymethylcellulose | 0,05 | |
| Polyacrylsäure | 0,10 | |
| Weizenstärke | 0,10 | |
| Magnesiumaluminiumsilikat | 0,50 | |
| Kaolin | 0,05 | |
| Talkum | 0,50 | |
| PEG-180/Laureth-50/TMMG Copolymer | 0,50 | |
| Parfüm, Konservierungsmittel, | | |
| Farbstoffe usw. | q.s. | |
| Natriumhydroxid | q.s. | |
| Wasser | ad 100,00 | |
| pH-Wert eingestellt auf 6,0-7,5 | | |
| Emulsion VI | | 35 |
| Gas (Argon) | | 65 |

Vordispergierung der anorganischen Gelbildner und Quellung der Hydrokolloide sowie des Polymers unter Rühren in der Wasserphase. Vereinigung der auf 78 °C aufgeheizten Fettphase mit der auf 75 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren im Becomix unter Begasung mit Argon bei 1 bar unter Kühlung auf 30 °C. Zugabe der Additive bei 30 °C (Parfüm, Wirkstoffe). Homogenisierung mittels einer Zahnkranzdispergiermaschine (Rotor-Stator-Prinzip) bei 23 °C.

| **Beispiel 7 (schaumförmige Sonnenschutz-Creme):** | | |
|---|---|---|
| Emulsion VII | Gew.-% | Vol.-% |
| Stearinsäure | 1,00 | |
| Cetylstearylalkohol | 4,00 | |
| Myristylalkohol | 1,00 | |
| Bornitrid | 1,00 | |
| Kaolin | 0,50 | |
| Silicadimethylsilylat | 1,50 | |
| PEG-20-Stearat | 1,00 | |
| Acrylat/C₁₀₋₃₀ Alkylacrylatcrosspolymer | 0,10 | |
| Hectorit | 0,20 | |
| Quaternium-18-Hectorit | 0.10 | |
| Caprylsäure/Caprinsäuretriglyceride | 2,00 | |
| Paraffinöl | 15,50 | |
| Dimethicon | 0,50 | |
| Octylisostearat | 5,00 | |
| Glycerin | 3,00 | |
| Octylmethoxycinnamat | 4,00 | |
| Butylmethoxydibenzoylmethan | 3,00 | |
| Ethylhexyltriazon | 3,00 | |
| PEG-180/Laureth-50/TMMG Copolymer | 1,50 | |
| BHT | 0,02 | |
| Na₂H₂EDTA | 0,10 | |
| Parfüm, Konservierungsmittel, | q.s. | |
| Farbstoffe, usw. | q.s. | |
| Kaliumhydroxid | q.s | |
| Wasser | ad 100,00 | |
| pH-Wert eingestellt auf 5,0-6,0 | | |
| Emulsion VII | | 35 |
| Gas (Helium) | | 65 |

Vordispergierung des anorganischen Gelbildners (Hectorite) und Quellung der Hydrokolloide sowie des Polymers unter Rühren in der Wasserphase. Vordispergierung des Quaternium-18-Hectorits in der heißen Fettphase. Vereinigung der auf 78 °C aufgeheizten Fett-/Lichtschutzfilterphase mit der auf 75 °C aufgeheizten Wasser-/Lichtschutzfilterphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren im Becomix unter Begasung mit Helium bei 1 bar unter Kühlung auf 30 °C. Zugabe der Additive bei 30 °C (Parfüm). Homogenisierung mittels einer Zahnkranzdispergiermaschine (Rotor-Stator-Prinzip) bei 23 °C.

## Patentansprüche

1. Selbstschäumende, schaumförmige, nachschäumende oder schäumbare kosmetische und dermatologische Zubereitungen, welche mindestens einen nicht-ionischen polymeren Verdicker gewählt aus der Gruppe der Copolymere aus
(a) Polyethylenglykol der Formel HO-CH₂(CH₂-O-CH₂)ₙ-CH₂-OH, worin n eine ganze Zahl von 100 bis 250 bedeutet,
(b) Polyoxyethylenglykolalkylether der Formel HO(CH₂CH₂O)ₓR¹, worin x eine ganze Zahl von 1 bis 100 und R¹ einen verzweigten oder unverzweigten, gesättigten oder ungesättigten Alkylrest mit 4 bis 40 Kohlenstoffatomen darstellen, und optional
(c) Tetramethoxymethylglycoluril
enthalten.

2. Verwendung eines oder mehrerer nicht-ionischer polymerer Verdicker gewählt aus der Gruppe der Copolymere aus
(a) Polyethylenglykol der Formel HO-CH₂(CH₂-O-CH₂)ₙ-CH₂-OH, worin n eine ganze Zahl von 100 bis 250 bedeutet,
(b) Polyoxyethylenglykolalkylether der Formel HO(CH₂CH₂O)ₓR¹, worin x eine ganze Zahl von 1 bis 100 und R¹ einen verzweigten oder unverzweigten, gesättigten oder ungesättigten Alkylrest mit 4 bis 40 Kohlenstoffatomen darstellen, und optional
(c) Tetramethoxymethylglycoluril
zur Schaumverstärkung selbstschäumender, schaumförmiger, nachschäumender oder schäumbarer kosmetischer und dermatologischer Zubereitungen.

3. Zubereitung nach Anspruch 1 oder Verwendung nach Anspruch 2, **dadurch gekennzeichnet, daß** Gesamtmenge des oder der Copolymeren aus dem Bereich von 0,01 bis 5 Gew.-%, bezogen auf das Gesamtgewicht der Zubereitung, gewählt wird.

4. Zubereitung nach Anspruch 1 oder Verwendung nach Anspruch 2, **dadurch gekennzeichnet, daß** der Volumenanteil des oder der Gase in der Zubereitung von 10 bis 80 Vol.-%, bezogen auf das Gesamtvolumen der Zubereitung, gewählt wird.

5. Zubereitung nach Anspruch 1 oder Verwendung nach Anspruch 2, **dadurch gekennzeichnet, daß** als Gas Kohlendioxid gewählt wird.

6. Zubereitung nach Anspruch 1 oder Verwendung nach Anspruch 2, **dadurch gekennzeichnet, daß** sie eine oder mehrere Substanzen, gewählt aus der Gruppe der Moisturizer, enthält.
